# EUROPEAN PATENT APPLICATION

(11) **EP 4 721 573 A1**
(43) Date of publication of application: **08.04.2026**
(21) Application number: 24383069.2
(22) Date of filing: 03.10.2024
(51) Int. Cl.: A23B 4/22, C12N 9/04, C12N 9/80, A01N 63/50

(54) **COMBINATION OF BACTERIOPHAGE-DERIVED ENDOLYSINS AND GLUCOSE OXIDASE AND USES THEREOF**

(71) Applicant: Consejo Superior De Investigaciones Científicas - CSIC, 28006 Madrid (ES); Universitat de València, 46010 Valencia (ES); Universitat Politécnica de Valencia, 46022 Valencia (ES)
(72) Inventor: Talens Perales, David, Valencia (ES); Darós Arnau, Jose Antonio, Valencia (ES); Polaina Molina, Julio, Valencia (ES); Marín Navarro, Julia Victoria, Valencia (ES)
(74) Representative: Pons IP

(57) **Abstract**

The present invention refers to a combination of enzymes that presents antimicrobial features. Specifically, the combination of the invention comprises an endolysin and a glucose oxidase. Surprisingly, the combination of the invention presents a synergistic antimicrobial effect that is useful for the control of food borne pathogens, such as *Listeria monocytogenes.*

## Description

The invention relates to a combination product that comprises enzymes and its uses as food preservative and antimicrobial agent in food. Thus, the present invention is within the biotechnology field, more specifically the invention is within the food biotechnology field.

### BACKGROUND ART

Poisoning caused by the ingestion of food infected by *Listeria monocytogenes* is a serious cause of concern worldwide. *L. monocytogenes* is a pathogen that can survive and grow under a wide range of rather extreme environmental conditions, including refrigeration temperatures and high salt concentrations. This resilience enables the bacterium to persist in various food processing environments and cause contamination. *L. monocytogenes* can contaminate a wide range of food products, including raw and processed meats, unpasteurized dairy products, fresh produce, and ready-to-eat foods. It can enter the food supply chain through various routes, such as soil, water, animals, and human carriers (Lakicevic B, et al. J Food Prot. 2023 Jan;86(1):100003). Contamination can occur during food production, processing, distribution, and handling. Because of the ubiquity of *Listeria,* risk assessment models advise the implementation of effective cleaning and sanitation measures all along the food chain (Gonzales-Barron U, et al. Foods. 2024 Jan 23;13(3):359).

Cleaning and disinfection constitute an essential activity in all food industries and business at all levels, from manufacturing to retail premises. This is true in general terms and particularly so in the case of resilient pathogens like *L. monocytogenes* (Tuytschaever T, et al. Compr Rev Food Sci Food Saf. 2023 Sep;22(5):3910-3950). Conventional disinfectants used in the food industry include chlorinated compounds, glutaraldehydes, alkylamines, alcohols and quaternary ammonia. Although effective, these compounds pose a serious environmental and health challenges as they can be highly toxic for humans.

Consequently, there is a need in the prior art to provide new alternative non-toxic, eco-friendly and effective antimicrobial agents that can be used in food industry.

### DESCRIPTION OF THE INVENTION

In view of the above-mentioned necessity, the present invention discloses a novel combination that comprises an endolysin and a glucose oxidase, wherein said combination has antimicrobial activity.

The combination provided by the present invention comprises active enzymes, therefore it is a more eco-friendly and better accepted alternative to the typical chemical compounds used in the food industry for antimicrobial purposes.

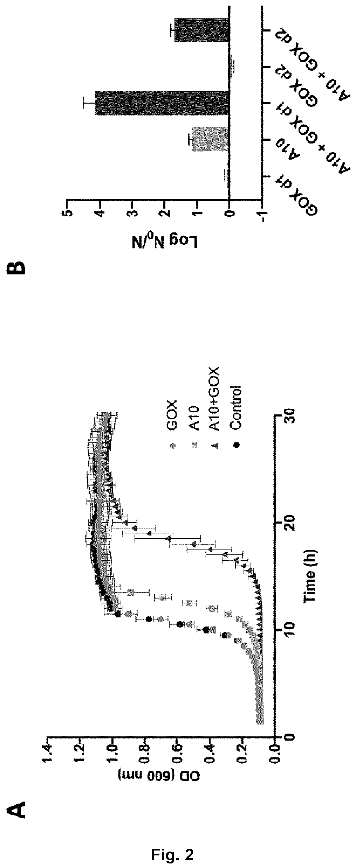

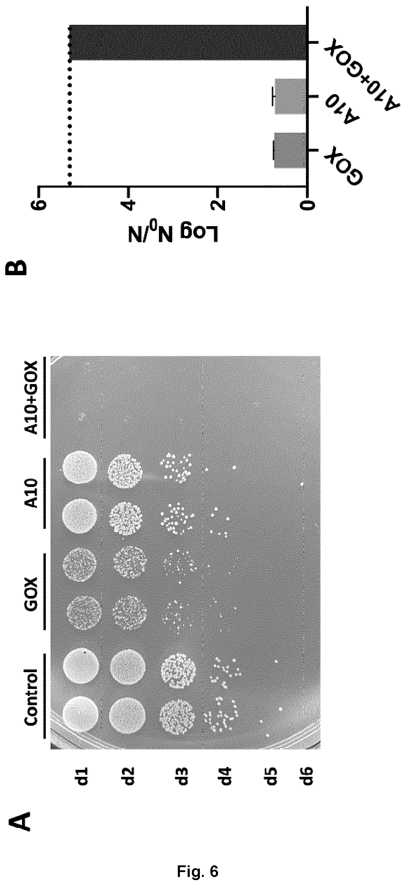

Moreover, as it is observed in the results provided by the present inventors, the combination of the invention shows a synergistic effect as antimicrobial (Fig.2), more interestingly it shows a synergistic antimicrobial effect against food borne bacteria belonging to *Listeria* such as *Listeria monocytogenes* (Fig.6), even at low doses.

Thus, one aspect of the present invention refers to a combination product, hereinafter the "combination of the invention", that comprises:
- an endolysin or a fragment thereof, and
- a glucose oxidase or a fragment thereof.

Hereinafter, the endolysin and the glucose oxidase comprised in the combination of the invention may also be referred to as the "endolysin of the invention" and the "glucose oxidase of the invention", respectively.

As it is observed in the results obtained by the present inventors, the endolysin and glucose oxidase showed some antimicrobial effect when used alone. However, when combined, the endolysin and a glucose oxidase surprisingly showed a synergistic effect as antimicrobial agent. In the present invention the terms "synergy" or "synergistic effect", refers to a situation in which the activity of a combination of an antimicrobial agent and at least one additional antimicrobial agent is greater than the sum of the activity of each agent when used individually.

As used in the present invention, the term "endolysin" refers to a bacteriophage-derived lytic enzyme that has the capability to degrade the bacterial cell-wall by cleaving the chemical links that bind molecules of the cell-wall.

In preferred embodiment, the endolysin of the invention is a N-acetylmuramoyl-L-alanine amidase which catalyses the chemical reaction that cleaves the link between the N-acetylmuramoyl residues and L-amino acid residues of the bacterial cell-wall. The enzyme classification number corresponding to N-acetylmuramoyl-L-alanine amidase is EC 3.5.1.28.

In another preferred embodiment, the endolysin of the invention comprises an amino acid sequence having at least 80% identity with SEQ ID NO: 1 or SEQ ID NO: 2.
SEQ ID NO: 1, amino acid sequence of N-acetylmuramoyl-L-alanine amidase of the phage vB_LmoS_188 (Genbank: AJE28029.1): 
MSVLQYNYINKNQFSRPGYKLLRVSKIVMHYTANPGASADNHRRYFRDLKERYAS
AHIFIDDNEAICIIPLNEVAYHANERSCKLTALQASTSYYRGGNANLTSIGIEMCLDKN
GNITAATFNRSVDVAAELCKTYDLTASDIIRHYDVTGKNCPAPWVAKPSELTRFRN
AVNAKLKGASQNKNRHDGKVVDSAPLLPKMDFKSSPFRMYKSGTEFLVYEHNQY
WYKTYINDKLYYMYKSFCDVVAKKDAKGRIPVKIKSAKDLRIPVWDNTRLNSGKIK
WYKPGTKLSWYDNKKGYLELWYDKDGWYYTANYFLK
SEQ ID NO: 2, amino acid sequence of N-acetylmuramoyl-L-alanine amidase of the phage PSU-VKH-LP041 (Genbank: AWN07992.1):

MATVNGVPFRQNLVSSSKYSIKAPNTMKAKKITIHNTYNNASSQNETDYCKNNNNE
VSFHVAVDDKEAIQVVPFGRNAWHCGDGGNGYGNRNTIGVETCYSKSGGAKYIA
AEKNTIKYVAGLCVQQDIIASKDTIKKHQDWSGKYCPHRILDDKRWPAVQQAIIDEY
NRITSKNPNRHSGQVVDSAPLLPKTDFKSNPARMYKSGTEFLVYEHNQYWYKTYI
DDKLYYMYKSFCDVVAKKDAKGRIKVRIKSAKDLRIPVWNNTKLSSGKIKWYKPGT
KLSWYNNKKGYLELWYTNDGWYYTANYFLK

In the present invention, "identity" or "sequence identity" is understood as the degree of similarity between two nucleotide or amino acid sequences obtained by optimal alignment of the two sequences. Depending on the number of common residues between the aligned sequences, a degree of identity expressed as a percentage will be obtained. The degree of identity between two amino acid sequences can be determined by conventional methods, for example, by standard sequence alignment algorithms known in the state of the art, such as BLAST. The BLAST programs, for example, BLASTN, BLASTX, and TBLASTX, BLASTP and TBLASTN, are in the public domain on the website of The National Center for Biotechnology Information (NCBI).

In another preferred embodiment, the endolysin of the combination of the invention comprises an amino acid sequence having at least 81, 82, 83, 84, 85, 86, 87, 88, 89, 90, 91, 92, 93, 94, 95, 96, 97, 98 or 99% identity with SEQ ID NO: 1 or SEQ ID NO: 2.

In another preferred embodiment, the endolysin of the combination of the invention comprises, or preferably consists of, the amino acid SEQ ID NO: 1 or SEQ ID NO: 2.

The combination product of the invention further comprises a glucose oxidase that exerts a synergistic antimicrobial effect with the endolysin. As used herein, the term "glucose oxidase" or "GOX", used interchangeably, refer to an enzyme that catalyses the oxidation of glucose to hydrogen peroxide and D-glucono-δ-lactone. The enzyme classification number corresponding to glucose oxidase is EC 1.1.3.4.

The use of an endolysin or a glucose oxidase independently has an antimicrobial effect that is less effective than the combination thereof. Thus, the combination of an endolysin and a glucose oxidase potentiates the antimicrobial effect, having further advantages since the use of a glucose oxidase as antimicrobial (alone) presents several limitations such as the high dose of the enzyme required to achieve an effective action and side effects due to the oxidative nature of the peroxide.

In a preferred embodiment, the glucose oxidase of the combination of the invention comprises an amino acid sequence having at least 80% identity with SEQ ID NO: 3.

SEQ ID NO: 3, amino acid sequence of Glucose oxidase (derived from Genbank AID16306.1 as described in Marin-Navarro J. et al. Plos One. 2015 Dec 7; 10: e014428):

SNGIEASLLTDPKDVSGRTVDYIIAGGGLTGLTTAARLTENPNISVLVIESGSYESDR
GPIIEDLNAYGDIFGSSVDHAYETVELATNNRTALIRSGNGLGGSTLVNGGTWTRP
HKAQVDSWETVFGNEGWNWDNVAAYSLQAERARAPNAKQIAAGHYFNASCHGT
NGTVHAGPRDTGDDYSPIVKALMSAVEDRGVPTKKDFGCGDPHGVSMFPNTLHE
DQVRSDAAREWLLPNYQRPNLQVLTGQYVGKVLLSQNGTTPRAVGVEFGTHKGN
THNVYAEHEVLLAAGSAVSPTILEYSGIGMKSILEPLGIDTVVDLPVGLNLQDQTTAT
VRSRITSAGAGQGQAAWFATFNETFGDYSEKAHELLNTKLEQWAEEAVARGGFH
NTTALLIQYENYRDWIVNHNVAYSELFLDTAGVASFDVWDLLPFTRGYVHILDKDPY
LHHFAYDPQYFLNELDLLGQAAATQLARNISNSGAMQTYFAGETIPGDNLAYDADL
SAWTEYIPEHFRPNYHGVGTCSMMPKEMGGVVDNAARVYGVQGLRVIDGSIPPM
QMSSHVMTVFYAMALKISDAILEDYASMQ

In another preferred embodiment, the glucose oxidase of the combination of the invention comprises an amino acid sequence having at least 81, 82, 83, 84, 85, 86, 87, 88, 89, 90, 91, 92, 93, 94, 95, 96, 97, 98 or 99% identity with SEQ ID NO: 3; preferably wherein the amino acid residue at positions 90, 509 and 554 is Arginine, Glutamic acid and Methionine, respectively.

In another preferred embodiment, the glucose oxidase of the combination of the invention comprises, or preferably consists of, the amino acid SEQ ID NO: 3.

The endolysin and glucose oxidase of the invention comprising an amino acid sequence having at least 80, 81, 82, 83, 84, 85, 86, 87, 88, 89, 90, 91, 92, 93, 94, 95, 96, 97, 98 or 99% identity with SEQ ID NO: 1 or SEQ ID NO: 2, or SEQ ID NO: 3, refer to functionally equivalent variants of the endolysins of SEQ ID NO: 1 or SEQ ID NO: 2, or glucose oxidase of SEQ ID NO: 3. Preferably, the term "functionally equivalent variant" in the present invention refers to a polypeptide or protein (in the present invention the protein is an enzyme) that has the same activity as a reference polypeptide or protein but that comprises an alteration in its sequence, that is, a substitution, insertion and/or deletion, in one or more (for example, several) positions. By way of example, a substitution means the replacement of the amino acid (occupying a position with a different amino acid; a deletion means the removal of the amino acid that occupies a position; and an insertion means adding an adjacent amino acid and immediately after the amino acid that occupies a position. The amino acids changes may be minor in nature, that is, conservative amino acid substitutions or insertions that do not significantly affect the folding and/or activity of the polypeptide/protein; small deletions, size 1-30 amino acids; small amino or carboxyl terminal extensions, such as an amino terminal methionine residue; a small linker peptide of up to 20-25 residues; or a small extension that facilitates purification by changing the net charge or other function, such as a polyhistidine tail, antigenic epitope, or binding domain. Examples of conservative substitutions are found within the groups of basic amino acids (arginine, lysine and histidine), amino acids (glutamic acid and aspartic), polar amino acids (glutamine and asparagine), hydrophobic amino acids (leucine, isoleucine and valine), aromatic amino acids (phenylalanine, tryptophan and tyrosine) and small amino acids (glycine, alanine, serine, threonine and methionine). Amino acid substitutions that generally do not alter specific activity are known in the art. Common examples of substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/lle, Leu/Val, Ala/Gly and Asp/Gly.

Moreover, the present invention also refers to the fragments of the endolysin or glucose oxidase of the invention. As used herein, the term "fragment" refers to a protein or polypeptide (such as the endolysin or glucose oxidase of the invention) with one or more amino acids absent from the ends of its sequence, such as the amino and/or carboxyl terminus of the amino acid sequence, preferably wherein said fragment keeps the same function as the complete protein or polypeptide. Therefore, the fragments of the endolysin of the invention or the glucose oxidase of the invention keep the same function as their complete enzymes. So that, the fragments of the endolysin or glucose oxidase of the invention are "functionally equivalent fragments".

Preferably, the fragments of the endolysin of the invention or the glucose oxidase of the invention lack at least 1, 2, 3 ,4 ,5, 6, 7, 8, 9, 10, 20, 30, 40, 50, 60, 70, 80, 90 or at least 100 amino acid residues from the amino and/or carboxyl terminus respect to the complete amino acid sequence of the endolysin or glucose oxidase of the invention.

More preferably, the fragments of the endolysin of the invention or the glucose oxidase of the invention lack from 1 to 140 amino acids, from 1 to 130 amino acids, 1 to 120 amino acids, from 1 to 110 amino acids, from 1 to 100 amino acids, from 1 to 90 amino acids, from 1 to 80 amino acids, from 1 to 70 amino acids, from 1 to 60 amino acids, from 1 to 50 amino acids, from 1 to 40 amino acids, from 1 to 30 amino acids, from 1 to 20 amino acids, from 1 to 10 amino acids and more preferably from 1 to 5 amino acid residues from the amino and/or carboxyl terminus respect to the complete amino acid sequence of the endolysin or glucose oxidase of the invention.

More preferably, the fragments of the endolysins of the invention lack from 1 to 150 amino acid residues form carboxyl terminus respect to the complete amino acid sequence of either endolysin of the invention, remaining intact the catalytic domain.

In another embodiment, the functionally equivalent fragment or functionally equivalent variants of the endolysin or glucose oxidase of the invention keep at least 60 %, more preferably 65, 70, 75, 80, 85, 90, 95, 96, 97, 98, 99 or 100 % of the enzymatic activity.

The enzymatic activity of the glucose oxidase of the invention may be determined as described in Marin-Navarro J. et al. Plos One. 2015 Dec 7; 10: e0144289. The enzymatic activity of the endolysin can be determined as described in Briers Y. et al. J Biochem Biophys Methods. 2007 Apr 10; 70(3): 531-3.

The endolysin and glucose oxidase of the invention can be produced, without limitation, by any synthesis or recombination method known for the skilled person, preferably the enzymes of the invention are recombinantly expressed in a host cell or tissue that comprises the nucleotide sequence coding thereof. The host cell may be a prokaryotic or eukaryotic cell, preferably a bacterial, animal, plant, fungal or yeast cell.

In the present invention, the endolysin and glucose oxidase are preferably "isolated" enzymes, which refers to a polypeptide or protein that has been identified and separated and/or recovered from a cell or cell culture from which it was expressed. No particular level of purification is required. For example, an isolated polypeptide can be removed from its native or natural environment. Recombinantly produced polypeptides and proteins expressed in host cells are considered isolated for purpose of the invention, as are native or recombinant polypeptides which have been separated, fractionated, or partially or substantially purified by any suitable technique. Examples of methods for isolating and/or purifying proteins or polypeptides include, without limitation, affinity chromatography, gel filtration, ion exchange chromatography or tagged protein purification.

In another preferred embodiment of the combination product of the invention, the endolysin of the invention and the glucose oxidase of the invention are comprised within the same composition or in separate compositions.

Preferably, the composition or compositions that comprise the endolysin and glucose oxidase of the invention are formulated as solid, liquid, gel or powder, preferably is formulated as a liquid composition or compositions.

In another preferred embodiment of the combination product of the invention, the endolysin and the glucose oxidase are comprised at an effective amount.

In another preferred embodiment, the endolysin of the invention is present at an amount from 0.1 µM to 40 µM and the glucose oxidase of the invention is at an amount from 0.05 U/mL to 8 U/mL, preferably in the same composition or in different compositions.

In another preferred embodiment, the endolysin of the invention is present at an amount from 10 µM to 30 µM and the glucose oxidase of the invention is at an amount from 2 U/mL to 8 U/mL, preferably in the same composition or in different compositions.

In the present invention, the term "U" refers to the unit of enzymatic activity as commonly known for the skilled person. A unit of enzymatic activity may be defined as the amount of enzyme that catalyzes the conversion of on micromole (µmol) of a substrate per unit time (minutes). So, the term "U/mL" refers to the units of enzymatic activity per millilitre of a composition.

In the present invention, with regard to the glucose oxidase, 1U refers to the amount of enzyme that oxidizes 1 µmol of glucose in 1 minute, preferably at 37 °C and pH 6, and more preferably determined as in Marin-Navarro J. et al. Plos One. 2015 Dec 7; 10: e0144289.

In another preferred embodiment, the composition or compositions that comprise the enzyme of the combination product of the invention, further comprise an acceptable excipient. In the present invention the term acceptable excipient refers to any substance that is added a composition to give consistency, shape, flavor, colour or other qualities that facilitate its dosage and/or activity, preferably the acceptable excipient does not lead to any interference on the activity of the combination of the invention. Examples of acceptable excipients include, without limitation diluents, coloring agents, thickeners or preservatives.

In another preferred embodiment, the combination of the invention further comprises glucose. The glucose helps the action of the glucose oxidase of the invention. More preferably, the glucose together with the endolysin and the glucose oxidase of the invention are within the same composition.

In a more preferred embodiment, the combination of the invention comprises from 0.5 to 4% (w/v) of glucose; preferably the combination of the invention comprises from 1 to 3 % (w/v) of glucose; more preferably the combination of the invention comprises 2% (w/v) of glucose. Preferably, the endolysin, glucose oxidase and glucose are comprised within the same composition.

As explained before, the combination product of the invention showed synergistic antimicrobial effect that is highly interesting in food industry.

Thus, in another aspect, the present invention refers to the use of the combination product of the invention as antimicrobial agent in food compositions, in food processing devices, in food processing facilities, on surfaces exposed to foods or in facilities used for the storage of food, hereinafter the "antimicrobial use of the invention".

As used herein, the term "antimicrobial agent" refers to a substance or composition that is able to kill or inhibit the growth of microbes, preferably bacteria.

The antimicrobial activity of the combination product of the invention is highly relevant in food industry since it allows to preserve the health and security standard requirements in food as such or devices or facilities that are in contact with foods.

As used in the present invention the "food composition" refers to a composition that comprises an edible ingredient wherein said composition can be consumed alone as food or can be added as an ingredient in other food products. The edible ingredient may comprise proteins, lipids, amino acids, carbohydrates, vitamins and/or combinations thereof. The food composition may be a processed food or unprocessed food, such as, but not limited to, meats, seafood products, fruits, vegetables, dairy products, cereals, legumes, eggs or milk.

Thus, in preferred embodiments, the food composition is selected from, meats, seafood products, fruits, vegetables, dairy products, cereals, legumes, eggs, milk or any composition comprising thereof.

In the present invention, the term "food processing device" refers to any device or machinery that is used in food processing or manufacturing, including, without limitation, devices such as crushers, grinders, blenders, marinating injectors or peelers. The "food processing facilities" or "facilities used for the storage of food" refers to facilities which include spaces where foods are directed to be processed or stored.

As used in the present invention, the term "surfaces exposed to foods" refer to the surface of any object that is in contact or may be in contact with foods, including, without limitation, the surface of a container or recipient that contains foods or the surfaces where foods are processed, for example a table.

Surprisingly, the present inventors found that the combination of the invention is highly effective against bacteria, more specifically against bacteria belonging to *Listeria* genus.

Thus, in a preferred embodiment of the antimicrobial use of the invention refers to the to the use of the combination product of the invention as antibacterial agent in food compositions, in food processing devices, in food processing facilities, on surfaces exposed to foods or in facilities used for the storage of food; more preferably as antimicrobial or antibacterial agent against belonging to genus *Listeria.*

In an even more preferred embodiment of the antimicrobial use of the invention, the bacteria belonging to listeria genus is *Listeria innocua* or *Listeria monocytogenes;* preferably *Listeria monocytogenes.*

*Listeria monocytogenes* is a microorganism that causes the food-borne disease named listeriosis that causes fever, muscle aches, nausea, vomiting, and diarrhea.

As described in Fig.6, the combination of the invention can inhibit *Listeria monocytogenes,* what can help to prevent the transmission of *Listeria* by foods.

In another aspect, the invention refers to the use of the combination of the invention as a food preservative.

The term "food preservative" refers, in the present invention, to a substance or combination of substances that is used to make foods last longer, preserve their organoleptic qualities and/or inhibit the growth of contaminant microorganisms. Therefore, food preservatives contribute to make foods safer and more attractive on a sensory level.

In another aspect, the present invention refers to a method for the microbial control that comprises applying the combination product of the invention with a food composition, a food processing device, food processing facilities, a surface exposed to foods or facilities used for the storage of food, hereinafter the "control method of the invention".

As used in the present invention, the term "microbial control" refers to reduction, inhibition, prevention of accumulation and/or elimination of microbes, such as bacteria.

The control method of the invention comprises applying the combination of the invention to the food or object where a microbial control is desired. The term "apply" or "applying" refers to the action of contacting or putting physically together the combination of the invention with a specific object (in the present invention, for example food or any object that is in contact with food).

In the control method of the invention, the applied combination of the invention preferably comprises the endolysin and the glucose oxidase within the same composition or in separate compositions.

In a preferred embodiment, the control method of the invention may comprise applying the endolysin and the glucose oxidase simultaneously or sequentially to the food composition, the food processing device, food processing facilities, the surface exposed to foods or facilities used for the storage of food.

The combination of the invention may be formulated in any suitable form for performing the microbial control in the control method of the invention.

In another preferred embodiment of the control method of the invention, the composition or compositions that comprise the endolysin and glucose oxidase of the invention are formulated as solid, liquid, gel or powder, preferably is formulated as a liquid composition or compositions. The application may be carried out by any method known for the skilled person, depending on the formulation, wherein such application may be liquid application, spray application or drenching.

Preferably, the combination of the invention is applied at an effective amount in the control method of the invention.

As used in the present invention, the term "effective amount" refers to the amount of the combination of the invention (i.e. the amount of endolysin and glucose oxidase) needed to produce the intended effect without producing any undesired effect. The intended effect in the present invention is the microbial control or the food preservation. As is evident to a person skilled in the art, the effective amount may vary depending on several factors such as, for example, how the combination is applied, the type of food or surface that the combination is connected to or the formulation of the combination. Undesired effects in the present invention may be the spoilage of food, changes in the organoleptic characteristics or any subsequent toxic effect caused in the person who ingests the food.

In another preferred embodiment of the control method of the invention refers to the microbial control of bacteria belonging to *Listeria*; preferably *Listeria monocytogenes.*

Another aspect of the present invention refers to a method for preserving a food composition that comprises applying the combination of the invention to the food composition, "hereinafter the preservation method of the invention".

In the preservation method of the invention, the applied combination of the invention comprises the endolysin and the glucose oxidase within the same composition or in separate compositions.

In a preferred embodiment, the preservation method of the invention may comprise applying the endolysin and the glucose oxidase simultaneously or sequentially to the food composition, the food processing device, food processing facilities, the surface exposed to foods or facilities used for the storage of food.

The combination of the invention may be formulated in any suitable form for performing the preservation of food.

In another preferred embodiment of the preservation method of the invention, the composition or compositions that comprise the endolysin and glucose oxidase of the invention are formulated as solid, liquid, gel or powder, preferably is formulated as a liquid composition or compositions. The application may be carried out by any method known for the skilled person, depending on the formulation, wherein such application may be liquid application, spray application or drenching.

Preferably, the combination of the invention is applied at an effective amount in the preservation method of the invention.

In another aspect, the present invention refers to a kit that comprises the combination product of the invention, hereinafter the "kit of the invention".

In preferred embodiment, the kit of the invention comprises the endolysin of the invention and the glucose oxidase of the invention comprised within the same composition or in separate compositions; preferably, wherein the composition or compositions that comprise the endolysin and glucose oxidase of the invention are formulated as solid, liquid, gel or powder, more preferably formulated as a liquid composition or compositions.

Thus, in a preferred embodiment, when the kit of the invention comprises the endolysin and the glucose oxidase in separate compositions, the kit of the invention is a kit of parts.

In addition, the kit of the invention may comprise other components useful in the implementation of the present invention, such as, buffers, substrates, material carriers, instructions or applicators.

In another aspect, the present invention refers to the use of the kit of the invention for food preservation.

Another aspect of the present invention refers to the use of the kit of the invention for microbial control in food compositions, in food processing devices, in food processing facilities, on surfaces exposed to foods or in facilities used for the storage of food.

In a preferred embodiment, the use of the kit of the invention is for microbial control of bacteria belonging to *Listeria*; preferably *Listeria monocytogenes.*

### DESCRIPTION OF THE DRAWINGS

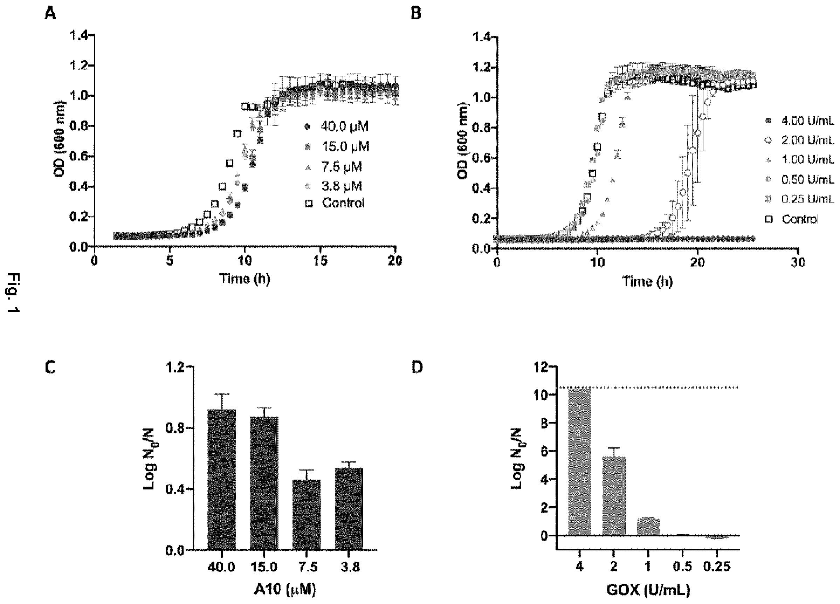

**Fig. 1****. Antimicrobial activity of A10 (A) and GOX (B) against *L. innocua.*** Log-phase cells were incubated with the enzybiotic at the expressed concentration for 2h (A10) or 90 minutes (GOX), or buffer (control). Afterwards, liquid medium was inoculated and growth was monitored spectrophotometrically (A, B). The log N₀/N ratio was quantified for each of treatments by comparison with the corresponding control (C, D). Error bars indicate the standard deviation of duplicates. The dotted line in D indicates the detection limit (10.4 Log N0/N).
**Fig. 2****. Synergistic antibacterial effect of A10 and GOX against *L. innocua.*** (A) Cells were treated with A10 (6 µM final concentration) for 30 minutes, followed by the addition of GOX (0.25 U/mL final concentration) and further incubation for 90 minutes. To assay the individual effect of either A10 or GOX, incubations were performed in the same conditions, replacing each of the enzymes with buffer. The control sample was prepared likewise replacing both enzymes with buffer. Subsequently, liquid medium was inoculated with cells subjected to the different treatments and growth was monitored spectrophotometrically. (B) The log N₀/N ratio was quantified for each treatment by comparison with the control using the same amidase concentration (15 µM) and two different GOX concentrations (d1 = 0.25 U/mL and d2 = 0.06 U/mL).

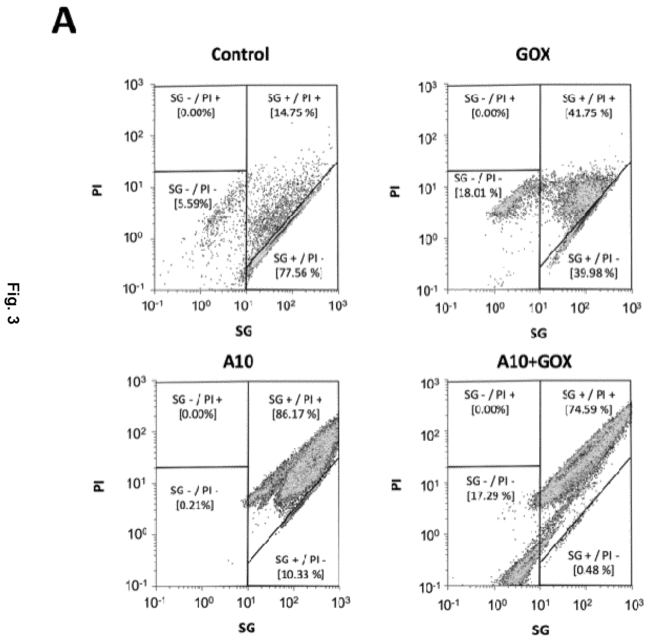

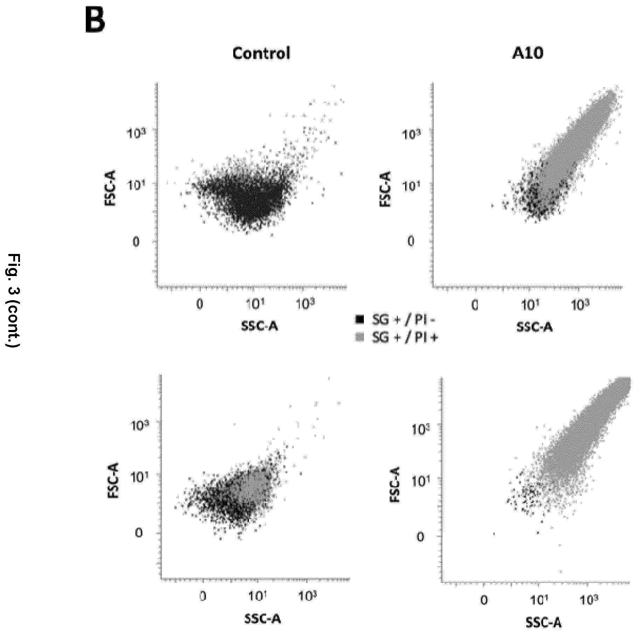

**Fig. 3****. Flow cytometry analysis of *L. innocua* cells treated with A10 amidase and GOX..** Cells were treated with A10 (15 µM final concentration) for 30 minutes, followed by the addition of GOX (0.25 U/mL final concentration) and further incubation for 90 minutes. A single, double and control treatment is shown. Panel A, SG/PI analysis of the whole sample in each case. Selected gates to discriminate SG +/- and PI +/populations are indicated. The percentage of cells in each gate is specified. Grey scale represents lower to higher events density. Panel B, SSC-A/FSC-A analysis of the SG + population. SG + / PI + and SG +/ PI- cells are labelled with different colors.; Forward Scatter Channel (FSC); Side Scatter Channel (SSC); Syto9 Acid Nucleic Stain (SG); Propidium Iodide (PI).

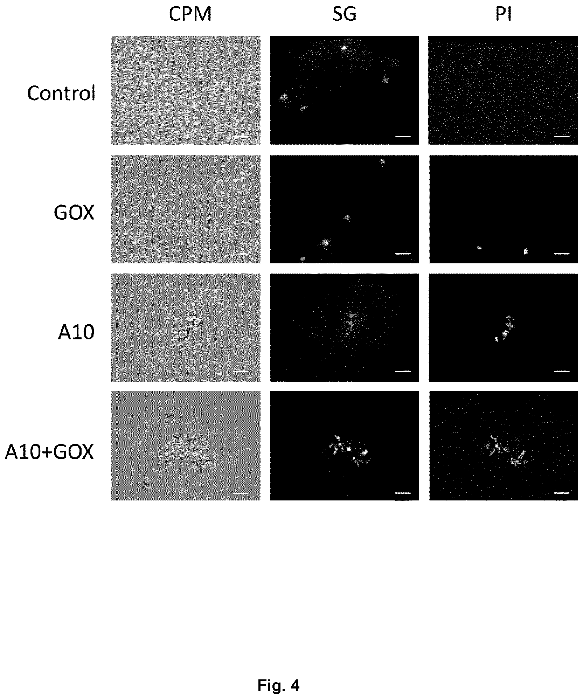

**Fig. 4****. Microscopy analysis of *L. innocua* after different treatments.** Bacterial cells were stained with Syto9 (SG) and propidium iodide (PI) fluorophores and observed at 1000X under phase contrast microscopy (CPM) and fluorescence with two different filters (SG and PI). Scale bars correspond to 5 µm.

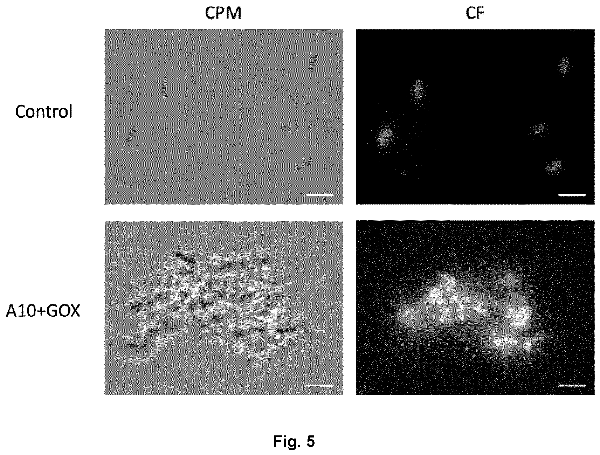

**Fig. 5****. Microscopy analysis of *L. innocua* after combined treatment with A10 and GOX.** Bacterial cells were stained with calcofluor (CF) and observed at 1000X under phase contrast microscopy (CPM) and fluorescence with UV-filter (CF). Arrows indicate putative carbohydrate fibers resulting from PG disassembly. Scale bars correspond to 10 µm.
**Fig. 6****. Synergistic antibacterial effect of A10 and GOX against L. monocytogenes.** Cells were treated with A10 (30 µM final concentration) for 30 minutes, followed by the addition of GOX (8 U/mL final concentration) and further incubation for 90 minutes. To assay the individual effect of either A10 or GOX, incubations were performed in the same conditions, replacing each of the enzymes with the corresponding buffer. The control sample was prepared likewise replacing both enzymes with buffer. Subsequently, dilutions d1 (1/10); d2 (1/100): d3 (1/10³); d4 (1/10⁴); d5 (1/10⁵) and d6 (1/10⁶) were carried out and spotted on LB-agar medium (A). The log N₀/N for each treatment was calculated from the reduction of colonies compared to the control treatment (B). The dashed line in B represents the detection limit of the method.

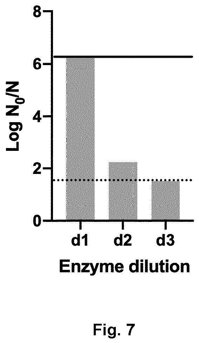

**Fig.7****. Non porous-surface test with A9 and GOX against *L. monocytogenes.*** An enzyme preparation containing 2 µM A9 endolysin, 3 U/mL GOX and 2 % glucose in buffer P (d1) with 0.3 g/L bovine serum albumin and two dilutions d2 (1/10) and d3 (1/10⁴) of the same preparation, were tested against *L. monocytogenes* on a non-porous surface for 45 minutes at 37 °C. The reduction in colony forming units on the disk surface after each treatment compared to a control with water was determined. The solid and dotted lines represent the upper and bottom detection limits of the method, respectively.

### Examples

In this section, experiments are provided showing the antimicrobial effect of a combination that comprises an endolysins and a glucose oxidase.

### 1. Material and methods

### 1.1 Enzyme production

The amino acid sequence of endolysin 188-amidase (henceforth A10, SEQ ID NO: 1) from *Listeria* phage vB_LmoS_188 (GenBank accession: AJE28029.1) and endolysin A9 (SEQ ID NO: 2, Genbank accession: AWN07992.1) from *Listeria* phage PSU-VKH-LP041 were reverse-transcribed to cDNA and edited for enhanced expression in *E. coli* efficiency using the Codon Optimization Tool provided by Integrated DNA Technologies (IDT). To facilitate cloning into expression vector pQE-80L (Qiagen), Sacl and Pstl restriction sites were incorporated at the 5' and 3' ends, respectively. Subsequently, the synthesized DNA fragment was digested with FastDigest Sacl and Pstl endonucleases (Thermo Scientific) and inserted into the cloning vector using T4 ligase (Thermo Scientific), to generate the gene plasmids pA9 and pA10. The endolysin coding region was fully sequenced to confirm the absence of mutations and pA9 or pA10 were used to transform competent Rosetta2 *E. coli* cells (Stratagene) for efficient protein production. Cell extracts were prepared from *E. coli* cultures grown at 37°C until an OD600 of 0.6. Gene induction was carried out by adding 1 mM IPTG followed by incubation at 16°C 16-48 h. Cells were disrupted by sonication in Buffer A (20 mM phosphate buffer, pH 7.4, 20 mM imidazole, 500 mM NaCl, 2% glycerol) and centrifuged at 12,000 g for 25 minutes. Purification of His-tagged endolysin was carried out by nickel affinity chromatography, employing a 1 mL HisTrap FF crude column (Cytiva) mounted on an AKTA-Purifier (Cytiva). Elution was performed with Buffer B (20 mM phosphate buffer, pH 7.4, 500 mM imidazole, 500 mM NaCl, 2% glycerol). Eluted fractions containing endolysin were dialyzed against buffer C (20 mM Tris-HCl, pH 7.0, 100 mM NaCl, 2% glycerol). Protein concentration in the eluted fractions was measured using a NanoDrop spectrophotometer (Thermo Fisher). Purity analysis of the recovered protein was evaluated by SDS-PAGE. Protein concentration in the eluted fractions was measured using a NanoDrop spectrophotometer (Thermo Fisher). The engineered version of glucose oxidase used in this work (henceforth GOX, SEQ ID NO: 3) was produced in *Nicotiana benthamiana,* extracted from the plant apoplast, and dialyzed against P buffer (50 mM sodium phosphate pH 6), as previously described (Talens-Perales et al 2023). Since GOX was not pure in these fractions, quantification of the enzyme was carried out by measuring GOX activity, expressed in mU/mL, where 1 unit represents the amount of enzyme that releases 1 µmol of H₂O₂ in 1 minute under the conditions of the assay. Activity assay was carried out as previously described (Marin-Navarro J, et al. PLoS One. 2015 Dec 7;10(12):e0144289).

### 1.2 Antibacterial assays

Antibacterial activity of A10 and GOX was assayed against *Listeria innocua* CECT 910T and *Listeria monocytogenes* CECT 940, type strains, which were obtained from the Spanish Type Culture Collection. Bacterial cultures were grown in liquid or solid TSB-YE media (Tryptic Soy Broth with 0.6% Yeast Extract) at 37°C to an OD of 0.6 at 600 nm. For assays in liquid medium, 300 µL of cells were collected by centrifugation at 12,000 g for 7 min, washed with 1 mL of buffer P and finally resuspended with the same buffer to a final optical density at 600 nm (OD₆₀₀) of 0.15 or 0.3.

Initially, cells were subjected to single treatments, either with amidase or GOX at different concentrations at 37°C. 80 µL of cells (OD₆₀₀ = 0.15) were either incubated with 80 µL of amidase for 2 h or with 80 µL of a GOX mix, containing glucose and GOX in buffer P, for 90 minutes. In all cases, glucose in the GOX mix was adjusted to a 2 % final concentration. Finally, a two-step treatment was carried out, in which 80 µL aliquots of cells (OD₆₀₀ = 0.15) were initially incubated with 20 µL of amidase A10 to a final concentration of 6 µM for 30 minutes at 37°C and subsequently, 60 µL of the GOX mix was added, followed by a 90 minutes incubation at 37°C. Control experiments were conducted by replacing the enzymes with buffer P in the appropriate volumes. A sample from each treatment was transferred to 300 µL of TSB-YE (TSB with 0.6 % yeast extract) in a 96-multiwell plate, resulting in a final 1/500 dilution, and incubated at 37°C. Growth was monitored by measuring the optical density (OD) at 600 nm every 30 minutes using a microplate reader (SPECTROstar Omega, BMG LABTECH). The growth curve was adjusted to a logistic model as previously described (Talens-Perales et al., 2023) to quantify the relative initial amount of cells after each treatment (N). By comparison with the relative initial amount of cells in the control treatment without enzybiotic (N₀), the log N₀/N was calculated. For assays on solid medium, 40 µL cells in buffer P (OD₆₀₀ = 0.3) were incubated with 20 µL of amidase at a final concentration of 30 µM, for 30 minutes at 37 °C, before the addition of 100 µL of GOX mix containing GOX at 8 U/mL (final concentration) and further incubated for 90 minutes at 37 °C. Serial dilutions (1/10) were carried out, 5 µL of each spotted on TSBYE-agar medium and grown at 37 °C for 16 h.

A quantitative test for the evaluation of bactericidal activity of disinfectants on non-porous surface was carried out. To this end, an enzyme preparation containing 2 µM A9 endolysin, 3 U/mL GOX and 2 % (w/v) Glucose in buffer P with 0.3 g/L bovine serum albumin was tested against *Listeria monocytogenes* (CECT 4031), without mechanical action, during 45 minutes at 37 °C under clean conditions, according to the EN 13697:2023 standard. In parallel, two dilutions of the enzymatic cocktail (1/10 and 1/10⁴) were also analysed. The surface test was composed of Discs of Steel iron 1.4301, 2 cm of diameter, 1.2 mm thick, and finishing grade 2B.

### 1.3 Flow cytometry

Control or treated cell samples were suspended in 160 µL of 0.8% NaCl, to which 40 µL of Syto9 Acid Nucleic Stain (SG) and Propidium Iodide (PI) was added to a final concentration of 6.7 µM and 40 µM, respectively, following instructions of the manufacturer (LIVE/DEAD BacLight bacterial viability kit, Invitrogen, Cat#L7012), and incubated for 15 minutes at room temperature before observation. Flow cytometry was conducted using a MACSQuant16 Analyzer (Miltenyi Biotec GmbH) equipped with MACSQuantify Software 2.13 (Miltenyi Biotec GmbH). The B1 channel (488 nm - 525/50) was employed to analyze the SG dye, while the B3 channel (488 nm - 615/20 nm) was used for the PI dye. The results obtained were analyzed using Floreada.io software (Floreada Devs). Gates were set manually based on the control sample. A threshold signal higher than 10 was fixed for SG positive (SG +) cells, covering 95 % of the control cells. The SG +/ PI - gate, corresponding to live cells, was established as a region corresponding to a relative lower incorporation of PI compared to SG within the SG + region, as previously described (Falcioni et al., 2008; Nescerecka et al., 2016). The complementary region was set as SG + / PI +, labelling dead cells.

### 1.4 Microscopy analysis.

For microscopy analysis, cells were stained using the same procedure as used for flow cytometry. Samples (5 µL) of the cells to be observed were placed onto a glass slide and covered with a 0.17 mm thick coverslip. Samples subjected to the two-step o treatment A10 and GOX or control samples incubated with buffer were additionally stained with 1 µL of calcofluor (1%) dye (SIGMA). Immersion oil type-a (mxa20234, Nikon) was applied over the coverslip before observation. Optical microscopy images were captured using an Eclipse 90i fluorescence microscope (Nikon) equipped with a 5-megapixel cooled digital color camera Nikon Digital Sight DS-5Mc (Nikon Corporation, Japan). Phase contrast illumination was employed, utilizing appropriate Nikon accessories (Ph3 condenser) and the CFI Plan Fluor DIC H/N2 100X Oil (MRH01900) objective. Fluorescent images were obtained using B-2E/C, G2-A, and UV2-A Nikon filter blocks for SG, PI, and CF, respectively using the same 100X objective. Images were processed by using FIJI image software.

### 2. Results

### 2.1 Synergistic biocidal effect of A10 amidase and GOX against Listeria innocua.

The antibacterial activity of A10 and GOX was measured independently as a first approach to establish the boundaries of experimental parameters. In order to study the dose-response of these treatments different concentrations were tested. In both cases, cell viability after incubation with each of the enzymes was monitored following the subsequent growth curve in a liquid medium (Fig. 1A and 1B). A quantitative analysis of the reduction in the number of initial cells after each treatment compared to the control (log N₀/N) indicated that the response range of the GOX treatment is wider than that of the amidase treatment (Fig. 1C and 1D). Concentrations equal or below the saturation limit of A10 (15 µM) were selected for subsequent experiments. In the case of GOX, in order to minimize hydrogen peroxide generation, subcritical doses (below 0.25 U/mL) were selected.

The combined effect of A10 and GOX was compared to the separate action of each enzyme (Fig. 2). In these experiments, a two-step treatment was carried out with a previous A10 incubation for 30 minutes at 6 µM, followed by a GOX addition at minimal dosis of 0.25 U/mL or 0.06 U/mL for 90 minutes. As shown in Fig. 2, the combined treatment with both enzybiotics caused a substantial decrease in bacterial viability as revealed by a significant growth delay (Fig. 2A). The log N₀/N ratio of the combined treatment showed a synergistic action compared to the single treatments (Fig. 2B).

### 2.2 Flow cytometry analysis of A10 amidase and GOX effect against Listeria

The effect of the two enzybiotics, either used separately or in combination, on cell morphology and integrity was studied by flow cytometry. Cell viability was analyzed by the double labelling method with SG/PI (Fig 3A). The population of live cells (SG +/PI -) was reduced from ca. 80 % in the control sample to 40 % and 10 % in the GOX and A10 single treatments, respectively. A synergistic effect was observed when both enzybiotics were combined, with a substantial reduction of live cells up to 0.5 %. These results support those obtained in the analysis of the log reduction of viable cells (log N₀/N) after single or combined treatments (Fig. 2B). Interestingly, when cells were incubated with GOX (either as a single or combined treatment), a significant increase in the SG - population was observed (Fig. 3A), which may be a consequence of a lower fluorophore stain due to DNA damage. FSC-A/SSC-A analysis showed a remarkable effect on cell morphology as a result of the treatment with A10 amidase, with or without GOX, which was mainly associated to the population of dead cells (Fig. 3B). The increase in FS, which is accompanied by an increase in SS, may be a result of cell aggregation as a consequence of peptidoglycan (PG) disassembly. This process was further studied by microscopy analysis.

### 2.3 Microscopy analysis of A10 amidase and GOX effect against Listeria

A microscopy study was conducted in order to confirm the morphology changes suggested by the flow-cytometry analysis. Only A10 treatments caused bacterial clumping (Fig. 4) and aggregates showed the double fluorophore labelling characteristic of dead cells. In the case of the double enzybiotic treatment the loss of individual cell morphology was more accused than with the single treatment with only A10. Calcofluor staining, which binds to cellulose and different β-glucans, including cellulose and chitin (Herth W & Sghnepf E. Protoplasma. 1980 Jul; 105:129-133; Mason DJ et al. J. Appl Bacteriol. 1995; 78:3-15; Perrine-Walker F & Payne J. Brazilian J. Microbiol. 2021; 52: 1077-1086; Wood PJ & Fulcher RG, The Journal of Histochemistny and Cytochemistry. 1983; 31(6): 823-826), was used to label the cell wall (Fig. 5). Since amidase cleaves the linkage between the polysaccharidic chain and the peptidic crosslink of the PG (Low LY et al., J. Biol. Chem. 2011; 286: 34391-34403), it would be feasible that enzymatic action releases carbohydrate fibers as those observed under the microscope (Fig. 5).

### 2.4 Validation of the combined effect of amidase and GOX against Listeria monocytogenes

The precedent sections clearly showed the antibacterial effect of A10 and GOX on *Listeria innocua* and the synergistic action resulting for the combination of both enzymes. Flow cytometry and microscopic analysis showed the lytic action of the enzymes on the bacteria, suggesting that a similar effect could be achieved against *Listeria monocytogenes.* That this is indeed the case is demonstrated by the result shown in Fig. 6. Single treatments with A10 or GOX resulted in a 0.7 log reduction, whereas the combination of both enzybiotics caused at least a 5.3 log reduction (i.e. over the detection limit of the method), even when a high cell density was used in the inoculum, confirming the synergistic effect of the treatment observed with *L. innocua.*

The synergistic effect of GOX with other amidases, specifically endolysin A9, was tested. The combination (GOX + A9), was applied on a previously *L. monocytogenes* inoculated non-porous surface and the reduction in colony forming units (CFUs) compared to a control with water was evaluated. The combination of A9 and GOX showed at least a 6.2 log reduction (beyond the detection limit of the method) against *L. monocytogenes* (Fig. 7), confirming the effectivity of this treatment.

The use of GOX as antimicrobial (alone) presents several limitations such as the high dose of the enzyme required to achieve an effective action, side effects due to the oxidative nature of the peroxide and the requirement of glucose for the enzyme reaction. In this study we show that the combination of A9 or A10 amidase, two endolysins produced by bacteriophages specific for *Listeria* and GOX, at fairly low dose of both enzymes, is highly effective for the elimination of the pathogen.

## Claims

1. A combination product that comprises:
- an endolysin or a fragment thereof, and
- a glucose oxidase or a fragment thereof.

2. The combination product according to claim 1, **characterized in that** the endolysin is a N-acetylmuramoyl-L-alanine amidase.

3. The combination product according to claim 1 or 2, **characterized in that** the endolysin comprises an amino acid sequence having at least 80% identity with SEQ ID NO: 1 or SEQ ID NO: 2 and/or the glucose oxidase comprises an amino acid sequence having at least 80% identity with SEQ ID NO: 3.

4. The combination product according to any one of claims 1 to 3, **characterized in that** the endolysin comprises the amino acid sequence SEQ ID NO: 1 or SEQ ID NO: 2 and/or the glucose oxidase comprises the amino acid sequence SEQ ID NO: 3.

5. The combination product according to any one of claims 1 to 4, **characterized in that** the endolysin and the glucose oxidase are comprised within the same composition or in separate compositions.

6. Use of the combination product according to any one of claims 1 to 5 as antimicrobial agent in food compositions, in food processing devices, in food processing facilities, on surfaces exposed to foods or in facilities used for the storage of food.

7. The use of the combination product according to claim 6, **characterized in that** combination product is used as an antimicrobial agent against bacteria belonging to genus *Listeria*; preferably *Listeria monocytogenes.*

8. Use of the combination product according to any one of claims 1 to 5 as a food preservative.

9. Method for the microbial control that comprises applying the combination product according to any one of claims 1 to 5 to a food composition, a food processing device, food processing facilities, a surface exposed to foods or facilities used for the storage of food.

10. The method according to claim 9 for microbial control of bacteria belonging to *Listeria;* preferably *Listeria monocytogenes.*

11. Method for preserving a food composition that comprises applying the combination product according to any one of claims 1 to 5 to the food composition.

12. A kit that comprises the combination product according to any one of claims 1 to 5.

13. Use of the kit according to claim 12 for food preservation.

14. Use of the kit according to claim 13 for microbial control in food compositions, in food processing devices, in food processing facilities, on surfaces exposed to foods or in facilities used for the storage of food.

15. The use of the kit according to claim 14 for microbial control of bacteria belonging to *Listeria;* preferably *Listeria monocytogenes.*
